# FASCICULE DE BREVET EUROPEEN

(11) **EP 0 433 135 B1**
(45) Date de publication et mention de la délivrance du brevet: **15.02.1995**
(21) Numéro de dépôt: 90403453.5
(22) Date de dépôt: 05.12.1990
(51) Int. Cl.: C07D 233/32, C08F 20/34, C07D 239/10, C07D 243/04, C07D 245/02, C07D 247/02

(54) **Procédé de préparation de (meth)acrylate d'alkylimidazolidone**
Verfahren zur Herstellung von Alkylimidazolidone(meth)acrylat
Process for the preparation of alkylimidazolidone (meth)acrylate

(30) Priorité: 15.12.1989 FR 8916607
(43) Date de publication de la demande: 19.06.1991
(73) Titulaire: ELF ATOCHEM S.A., 92800 Puteaux (FR)
(72) Inventeur: Grosius, Paul, F-57540 Petite-Rosselle (FR); Vanhoye, Didier, F-57600 Forbach (FR)

(56) Documents cités:
- EP-A- 0 124 713
- EP-A- 0 236 994
- FR-A- 2 192 104
- US-A- 2 871 223

## Description

La présente invention se rapporte à un nouveau procédé de synthèse des acrylates et méthacrylates d'alkylimidazolidone.

Les acrylates et méthacrylates d'alkylimidazolidone sont connus pour leur rôle dans la constitution de polymères utiles comme revêtements et adhésifs, et pour le traitement du papier et des textiles, notamment par le brevet US-A-2 871 223, ainsi que pour leur utilisation comme agents de traitement du cuir et dans la production de peintures en émulsion. On manquait toutefois jusqu'à présent d'un procédé de synthèse sélectif et aisément transposable à l'échelle industrielle pour ces produits. Un tel procédé constitue donc l'objectif de la présente invention.

L'objet de la présente invention consiste donc en un procédé de préparation d'un composé de formule : 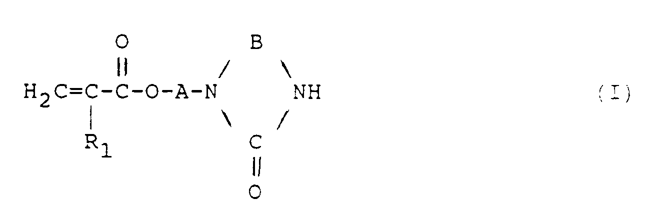
dans laquelle :
- R₁ est choisi parmi l'hydrogène et le groupe méthyle, et
- A et B sont choisis parmi les groupes alkylène à chaîne droite ou ramifiée ayant de 2 à 5 atomes de carbone,
par réaction d'au moins un (méth)acrylate de formule : 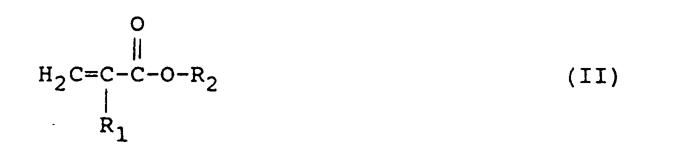
dans laquelle R₁ a la signification précitée et R₂ est un radical alkyle possédant de 1 à 4 atomes de carbone, avec un alcool hétérocyclique de formule 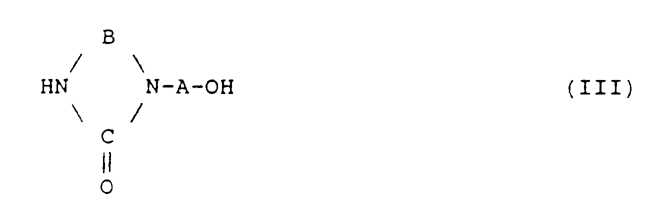
dans laquelle A et B ont la signification précitée. Un tel procédé a déjà été décrit par EP A 0 236 994, il a lieu en présence d'alcoolates de titane ou de chélates de titane, zirconium, fer et zinc avec des compris 1,3-dicarbonyle. Conformément à la présente invention, il a lieu en présence d'un catalyseur choisi parmi les dialkyloxydes d'étain, les dialkyldialcoxydes d'étain et les dialkyldiesters d'étain.

Comme exemples de réactifs de formule (II), on peut citer notamment les acrylates et méthacrylates de méthyle, d'éthyle, de n-propyle, d'isopropyle, de n-butyle, d'isobutyle et de terbutyle et leurs mélanges en toutes proportions. Comme exemple d'alcool hétérocyclique de formule (III), on peut citer notamment la 1-(2-hydroxyéthyl) imidazolidyl-2-one. Comme exemples de catalyseur, on peut citer notamment le di-n-butyl oxyde d'étain, le di-n-octyl oxyde d'étain, le bis di-n-butyl acétoxyétain et leurs mélanges en toutes proportions.

La quantité de catalyseur utilisée pour la mise en oeuvre du procédé selon l'invention est généralement comprise entre 0,05% et 2% environ en moles, de préférence entre 0,1% et 1% en moles par mole d'alcool hétércoyclique de formule (III).

La réaction du procédé selon l'invention peut être effectuée en présence d'un excès de l'un ou l'autre des réactifs. Il est toutefois conseillé que le rapport molaire (méth)acrylate de formule (II)/alcool hétérocyclique de formule (III) soit compris entre 1,1 et 6,0 environ, de préférence entre 2,0 et 5,0. En opérant avec un large excès molaire de (méth)acrylate par rapport à l'alcool hétérocyclique, on obtiendra à l'issue de la réaction une solution de composé de formule (I) dans le (méth)acrylate qui peut être utilisée directement pour certaines applications telles que l'obtention de peintures et revêtements ou bien le traitement du cuir.

La réaction du procédé selon l'invention est de préférence effectuée en présence d'au moins un inhibiteur de polymérisation, utilisé par exemple à raison de 0,05% à 0,5% en poids sur la base du poids de l'alcool hétérocyclique de formule (III). Comme exemples d'inhibiteurs de polymérisation utilisables, on peut citer notamment la phénothiazine, l'éther méthylique de l'hydroquinone, la N,N-diéthylhydroxylamine, le nitrobenzène, le di-tertiobutylcatéchol, l'hydroquinone, le p-anilinophénol, le phosphite de di-(2-éthylhexyl)-octylphényle et leurs mélanges en toutes proportions.

La réaction du procédé selon l'invention est effectuée de préférence sous une pression ne dépassant pas la pression atmosphérique, par exemple une pression comprise entre 3.10⁴-10⁵ Pa (0,3 et 1 bar). Elle est effectuée en mélangeant le (méth)acrylate de formule (II) et l'alcool hétérocyclique de formule (III) et en chauffant le mélange réactionnel au reflux total, généralement à une température comprise entre 70°C et 120°C environ, de préférence entre 80°C et 110°C, cette température étant évidemment dépendante de la nature exacte de l'alcool et du (méth)acrylate.

Dans la mise en oeuvre du procédé selon l'invention, il est conseillé d'atteindre une déshydratation maximale avant l'addition du catalyseur, de manière à éviter la désactivation de ce dernier par l'eau. On peut atteindre ce résultat par exemple en chauffant le mélange initial de (méth)acrylate de formule (II), d'alcool hétérocyclique de formule (III) et le cas échéant d'inhibiteur de polymerisation au reflux total puis en séparant par distillation l'azéotrope de (méth)acrylate et d'eau. A ce stade, après séparation du distillat et après refroidissement du mélange réactionnel suffisant pour condenser toutes les vapeurs qui se trouvent dans la colonne, le catalyseur peut être introduit dans le mélange réactionnel.

La durée de la réaction selon l'invention, dépendant évidemment de conditions réactionnelles telles que la température, la pression et la quantité de catalyseur utilisé, est généralement comprises entre 1 et 10 heures environ.

Le mélange réactionnel est donc chauffé au reflux total jusqu'à ce que la température de tête atteigne la température de distillation de l'azéotrope du (méth)acrylate et de l'alcool de formule R₂OH formé par la réaction. Cette température de distillation est de 65°C environ en ce qui concerne l'azéotrope de méthanol et de méthacrylate de méthyle, 82°C environ en ce qui concerne l'azéotrope d'éthanol et de méthacrylate d'éthyle, et 84°C environ en ce qui concerne l'azéotrope d'éthanol et d'acrylate d'éthyle. Quels que soient les composés présents dans la colonne, la température de tête doit être maintenue en-dessous de 120°C environ, au besoin en utilisant une pression réduite, pour éviter tout risque de polymérisation. En général, il est désirable d'abaisser progressivement la pression après la séparation par distillation de l'azéotrope d'alcool formé par la réaction et de (méth)acrylate.

L'excès éventuel de (méth)acrylate peut ensuite être éliminé par évaporation (opération dite de strippage), de manière à isoler le composé de formule (I) du milieu réactionnel, généralement à l'état solide : ainsi l'acrylate de 1-(2-hydroxyéthyl)imidazolidyl-2-one est un solide blanc cristallin de température de fusion égale à 43°C, soluble à froid dans les cétones, les alcools, les hydrocarbures aromatiques et l'eau, insoluble à froid dans les hydrocarbures saturés et qui précipite à 0°C dans l'acrylate d'éthyle. Le méthacrylate de 1-(2-hydroxyéthyl) imidazolidyl-2-one est un solide blanc cristallin de température de fusion égale à 47°C, possédant les mêmes propriétés de solubilité que le précédent. A l'issue de l'opération de strippage, le produit solide cristallin peut en outre être purifié par lavage par un alcool léger tel que le méthanol et/ou par un éther de pétrole, puis filtration et séchage.

L'isolement du composé de formule (I) peut aussi être réalisé par strippage partiel du (méth)acrylate pius cristallisation à une température suffisamment basse (de préférence inférieure ou égale à 0°C) et pendant une durée suffisamment longue (pouvant atteindre jusqu'à 15 heures), puis filtration suivie des étapes de purification décrites ci-dessus.

Enfin, une troisième méthode pour isoler le composé de formule (I) de la solution le contenant consiste à effectuer une extraction par l'eau, suivie d'une décantation, d'un strippage du (méth)acrylate et des étapes de purification décrites ci-dessus.

Ainsi le procédé selon l'invention permet d'obtenir, soit pur soit sous forme de solution dans le (méth)acrylate, le composé de formule (I) avec un rendement élevé : dans le cas du méthacrylate de 1-(2-hydroxyéthyl) imidazolidyl-2-one, le rendement dépasse couramment 94% et peut atteindre jusqu'a 99%. Dans le cas de l'acrylate de 1-(2-hydroxyéthyl)imidazolidyl-2-one, le rendement dépasse couramment 80% et peut atteindre jusqu'a 88%.

Les exemples suivants sont donnés à titre illustratif de la présente invention.

### EXEMPLE 1

Dans un réacteur de 0,5 litre fonctionnant sous pression atmosphérique, on introduit 450 g de méthacrylate de méthyle stabilisé par 250 ppm d'éther méthylique de l'hydroquinone, 130 g de 1-(2-hydroxyéthyl)imidazolidyl-2-one, 1,24 g de di-n-butyl oxyde d'étain et 0,2 g de phénothiazine. Le mélange réactionnel est chauffé jusqu'a ébullition. Le mélange, d'abord hétérogène, devient translucide au bout d'une heure tandis que l'azéotrope de méthanol et de méthacrylate de méthyle distille en tête à la température de 65°C. Après 5 heures 10 minutes de réaction, on diminue progressivement la pression jusqu'à 6.10⁴ Pa (0,6 bar). Le contenu du récteur est ensuite refroidi à température ambiante puis vidangé. Le rendement molaire en méthacrylate de 1-(2-hydroxyéthyl)imidazolidyl-2-one, déterminé par dosage par chromatographie liquide haute performance, est égal à 98,4%. On obtient ainsi une solution de méthacrylate de 1-(2-hydroxyéthyl)imidazolidyl-2-one à une concentration de 40% environ dans le méthacrylate de méthyle, à partir de laquelle on peut isoler le produit réactionnel si désiré.

### EXEMPLE 2

On reproduit la procédure expérimentale de l'exemple 1, sauf que l'on remplace le di-n-butyl oxyde d'étain par 3,64 g de bisdibutylacétoxyde d'étain (commercialisé par la société M & T CHIMIE), correspondant à 0,6% molaire par rapport à l'alcool, et que la réaction est poursuivie pendant 6 heures et 30 minutes. Le rendement molaire en méthacrylate de 1-(2-hydroxyéthyl)imidazolidyl-2-one est égal à 96,7%.

### EXEMPLE 3

Dans un réacteur de 0,5 litre fonctionnant sous pression atmosphérique, on introduit 450 g d'acrylate d'éthyle stabilisé par 250 ppm d'éther méthylique de l'hydroquinone, 130 g de 1-(2-hydroxyéthyl)imidazolidyl-2-one, 2,48 g de di-n-butyl oxyde d'étain et 0,5 g de phénothiazine. Le mélange réactionnel est chauffé jusqu'a ébullition. Le mélange, d'abord hétérogène, devient translucide au bout d'une heure tandis que l'azéotrope d'éthanol et d'acrylate d'éthyle distille en tête à la température de 84°C. Après 7 heures de réaction, on diminue progressivement la pression jusqu'à 6.10⁴ (0,6 bar). Le contenu du réacteur est ensuite refroidi à température ambiante puis vidangé. Le rendement molaire en acrylate de 1-(2-hydroxyéthyl)imidazolidyl-2-one, déterminé par dosage par chromatographie liquide haute performance, est égal à 86%. On obtient ainsi une solution d'acrylate de 1-(2-hydroxyéthyl)imidazolidyl-2-one à une concentration de 33% environ dans l'acrylate d'éthyle, à partir de laquelle on peut isoler le produit réactionnel si désiré.

## Revendications

1. Procédé de préparation d'un composé de formule: 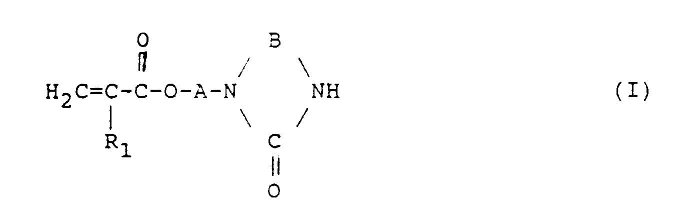 dans laquelle :
- R₁ est choisi parmi l'hydrogène et le groupe méthyle, et
- A et B sont choisis parmi les groupes alkylène à chaîne droite ou ramifiée ayant de 2 à 5 atomes de carbone,
par réaction d'au moins un (méth)acrylate de formule : 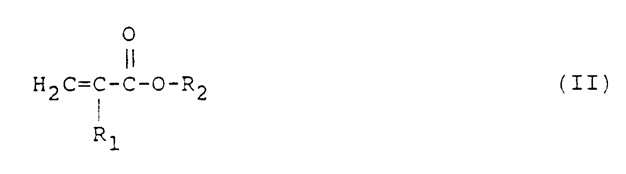 dans laquelle R₁ a la signification précitée et R₂ est un radical alkyle possédant de 1 à 4 atomes de carbone, avec un alcool hétérocyclique de formule 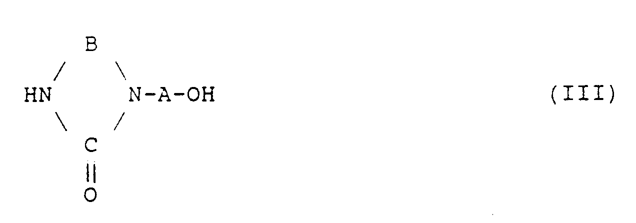 dans laquelle A et B ont la signification précitée, en présence d'au moins un catalyseur choisi parmi les dialkyloxydes d'étain, les dialkyldialcoxydes d'étain et les dialkyldiesters d'étain.

2. Procédé selon la revendication 1, caractérisé en ce que le catalyseur est choisi parmi le di-n-butyl oxyde d'étain, le di-n-octyl oxyde d'étain, le bis-di-n-butyl acétoxyétain et leurs mélanges en toutes proportions.

3. Procédé selon l'une des revendications 1 et 2, caractérisé en ce que le catalyseur est utilisé en quantité comprise entre 0,05% et 2% en moles par mole d'alcool hétérocyclique de formule (III).

4. Procédé selon l'une des revendications 1 à 3, caractérisé en ce que le rapport molaire (méth)acrylate de formule (II)/alcool hétérocyclique de formule (III) est compris entre 1,1 et 6,0.

5. Procédé selon l'une des revendications 1 à 4, caractérisé en ce que la réaction est effectuée en présence d'au moins un inhibiteur de polymérisation.

6. Procédé selon la revendication 5, caractérisé en ce que l'inhibiteur de polymérisation est choisi parmi la phénothiazine, l'éther méthylique de l'hydroquinone, la N,N-diéthylhydroxylamine, le nitrobenzène, le di-tertiobutylcatéchol, l'hydroquinone, le p-anilinophénol, le phosphite de di-(2-éthylhexyl)-octylphényle et leurs mélanges en toutes proportions.

7. Procédé selon l'une des revendications 5 et 6, caractérisé en ce que l'inhibiteur de polymérisation est utilisé à raison de 0,05% et 0,5% en poids sur la base du poids de l'alcool hétérocyclique de formule (III).

8. Procédé selon l'une des revendications 1 à 7, caractérisé en ce que la durée de la réaction est comprise entre 1 et 10 heures.

9. Procédé selon l'une des revendications 1 à 8, caractérisé en ce que la réaction est effectuée sous une pression ne dépassant pas la pression atmosphérique.

10. Procédé selon l'une des revendications 1 à 9, caractérisé en ce que la réaction est effectuée à une température comprise entre 70°C et 120°C.

## Claims

1. Process for the preparation of a compound of formula: 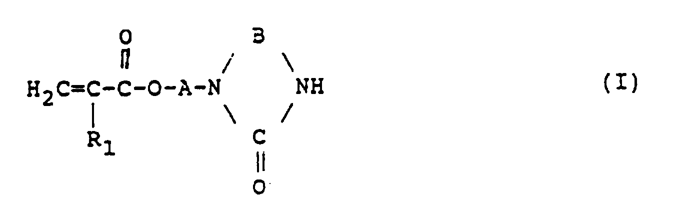 in which :
- R₁ is chosen from hydrogen and the methyl group, and
- A and B are chosen from straight- or branched-chain alkylene groups having from 2 to 5 carbon atoms,
by reaction of at least one (meth)acrylate of formula : 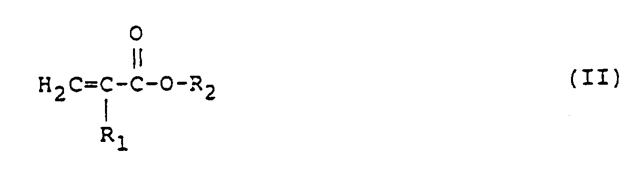 in which R₁ has the abovementioned meaning and R₂ is an alkyl radical having from 1 to 4 carbon atoms, with a heterocyclic alcohol of formula 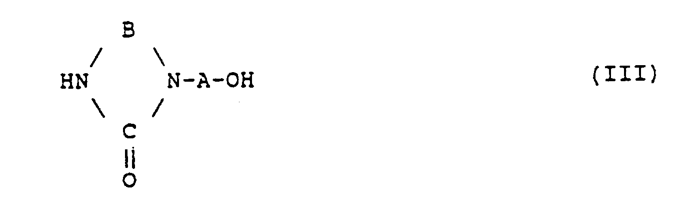 in which A and B have the abovementioned meaning, in the presence of at least one catalyst chosen from dialkyltin oxides, dialkyltin dialkoxides and dialkyltin diesters.

2. Process according to Claim 1, characterized in that the catalyst is chosen from di(n-butyl)tin oxide, di(n-octyl)tin oxide, di(n-butyl)tin diacetate and their mixtures in all proportions.

3. Process according to either of Claims 1 and 2, characterized in that the catalyst is used in an amount of between 0.05 and 2 mol % per mole of heterocyclic alcohol of formula (III).

4. Process according to one of Claims 1 to 3, characterized in that the (meth)acrylate of formula (II)/heterocyclic alcohol of formula (III) molar ratio is between 1.1 and 6.0.

5. Process according to one of Claims 1 to 4, characterized in that the reaction is carried out in the presence of at least one polymerization inhibitor.

6. Process according to Claim 5, characterized in that the polymerization inhibitor is chosen from phenothiazine, the methyl ether of hydroquinone, N,N-diethylhydroxylamine, nitrobenzene, di(tert-butyl)catechol, hydroquinone, p-anilinophenol, di(2-ethylhexyl) octylphenyl phosphite and their mixtures in all proportions.

7. Process according to either of Claims 5 and 6, characterized in that the polymerization inhibitor is used in a proportion of 0.05 to 0.5 weight % based on the weight of the heterocyclic alcohol of formula (III).

8. Process according to one of Claims 1 to 7, characterized in that the duration of the reaction is between 1 and 10 hours.

9. Process according to one of Claims 1 to 8, characterized in that the reaction is carried out under a pressure not exceeding atmospheric pressure.

10. Process according to one of Claims 1 to 9, characterized in that the reaction is carried out at a temperature of between 70°C and 120°C.

## Patentansprüche

1. Verfahren zur Herstellung von Verbindungen der Formel 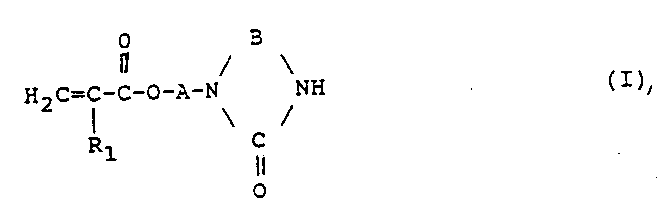 in der
- R₁ unter Wasserstoff und Methyl und
- A und B unter geradkettigen oder verzweigten Alkylengruppen mit 2 bis 5 Kohlenstoffatomen
ausgewählt sind, bei dem mindestens ein Methacrylat der Formel 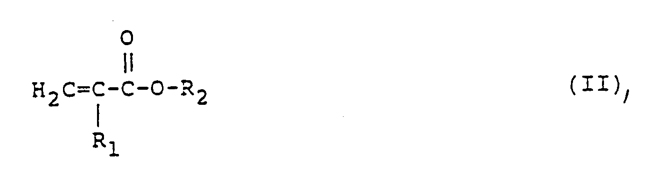 in der R₁ die obengenannte Bedeutung hat und R₂ eine Alkylgruppe darstellt, die 1 bis 4 Kohlenstoffatome aufweist,
mit einem heterocyclischen Alkohol der Formel 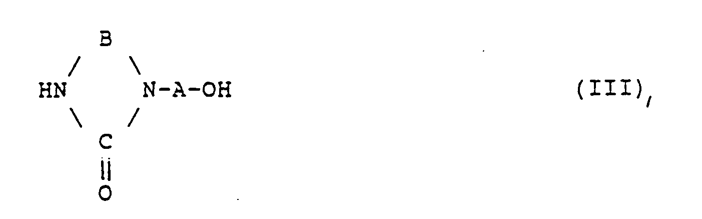 in der A und B die obengenannte Bedeutung haben, in Gegenwart von mindestens einem Katalysator umgesetzt wird, der unter Dialkylzinnoxiden, Dialkyldialkoxy-Zinnverbindungen und Dialkyldiester-Zinnverbindungen ausgewählt ist.

2. Verfahren nach Anspruch 1, dadurch gekennzeichnet, daß der Katalysator unter Di-n-butylzinnoxid, Di-n-octylzinnoxid und bis-Di-n-butylacetoxyzinn und beliebig zusammengesetzten Gemischen davon ausgewählt ist.

3. Verfahren nach einem der Ansprüche 1 und 2, dadurch gekennzeichnet, daß der Katalysator in einer Menge von 0,05 bis 2 Mol-% pro Mol heterocyclischen Alkohols der Formel (III) verwendet wird.

4. Verfahren nach einem dem Ansprüche 1 bis 3, dadurch gekennzeichnet, daß das molare Verhältnis des Methacrylats der Formel (II) zum heterocyclischen Alkohol der Formel (III) 1,1 bis 6,0 beträgt.

5. Verfahren nach einem der Ansprüche 1 bis 4, dadurch gekennzeichnet, daß die Reaktion in Gegenwart von mindestens einem Polymerisationsinhibitor durchgeführt wird.

6. Verfahren nach Anspruch 5, dadurch gekennzeichnet, daß der Polymerisationsinhibitor ausgewählt ist unter Phenothiazin, Hydrochinonmethylether, N,N-Diethylhydroxylamin, Nitrobenzol, Di-tert.-butylbrenzcatechin, Hydrochinon, p-Anilinophenol, Di-(2-ethylhexyl)octylphenylphosphit und beliebig zusammengesetzten Gemischen davon.

7. Verfahren nach einem der Ansprüche 5 und 6, dadurch gekennzeichnet, daß der Polymerisationsinhibitor in einem Verhältnis von 0,05 bis 0,5 Gew.-%, bezogen auf das Gewicht des heterocyclischen Alkohols der Formel (III), verwendet wird.

8. Verfahren nach einem der Ansprüche 1 bis 7, dadurch gekennzeichnet, daß die Umsetzung 1 bis 10 h dauert.

9. Verfahren nach einem der Ansprüche 1 bis 8, dadurch gekennzeichnet, daß die Umsetzung bei einem Druck durchgeführt wird, der den Atmosphärendruck nicht übersteigt.

10. Verfahren nach einem der Ansprüche 1 bis 9, dadurch gekennzeichnet, daß die Reaktion bei einer Temperatur von 70 bis 120 °C durchgeführt wird.
